# EUROPÄISCHE PATENTSCHRIFT

(11) **EP 4 321 888 B1**
(45) Veröffentlichungstag und Bekanntmachung des Hinweises auf die Patenterteilung: **11.03.2026**
(21) Anmeldenummer: 22190144.0
(22) Anmeldetag: 12.08.2022
(51) Int. Cl.: G01R 33/422, G01R 33/563

(54) **MAGNETRESONANZ-ELASTOGRAPHIE-VORRICHTUNG**
MAGNETIC RESONANCE ELASTOGRAPHY DEVICE
DISPOSITIF D'ÉLASTOGRAPHIE PAR RÉSONANCE MAGNÉTIQUE

(43) Veröffentlichungstag der Anmeldung: 14.02.2024
(73) Patentinhaber: Siemens Healthineers AG, 91301 Forchheim (DE)
(72) Erfinder: Höcht, Philipp, 91207 Lauf (DE); Stich, Manuel, 92711 Parkstein (DE)
(74) Vertreter: Siemens Healthineers Patent Attorneys

(56) Entgegenhaltungen:
- EP-A1- 3 736 594
- JP-A- 2002 010 991
- US-A1- 2015 148 663
- WOLFGANG LOEW ET AL: "A Dedicated 3 Tesla Prostate Coil for Magnetic Resonance Elastography, Imaging, and Tracking", PROCEEDINGS OF THE INTERNATIONAL SOCIETY FOR MAGNETIC RESONANCE IN MEDICINE, ISMRM, JOINT ANNUAL MEETING ISMRM-ESMRMB, MILAN, ITALY, 10-16 MAY 2014, no. 2180, 25 April 2014 (2014-04-25), XP040663249
- QITE CHEN ET AL: "The Design of Shear Wave Drive Device in Magnetic Resonance Elastography", BIOINFORMATICS AND BIOMEDICAL ENGINEERING, (ICBBE) 2011 5TH INTERNATIONAL CONFERENCE ON, IEEE, 10 May 2011 (2011-05-10), pages 1 - 4, XP031878910, ISBN: 978-1-4244-5088-6, DOI: 10.1109/ICBBE.2011.5780456

## Beschreibung

Die vorliegende Erfindung betrifft eine Magnetresonanz-Elastographie-Vorrichtung mit einer Magnetresonanzvorrichtung und einer Elastographievorrichtung.

Tumorgewebe und gesundes Gewebe, insbesondere Gewebe, das tumorfrei ist, weisen unterschiedliche Vibrationseigenschaften und/oder ein unterschiedliches Anregungsverhalten bei einer Vibrationsanregung auf. Die diagnostische Bildgebung nutzt bei der Elastographie die unterschiedlichen Vibrationseigenschaften und/oder das unterschiedliche Anregungsverhalten zwischen den unterschiedlichen Gewebearten, insbesondere einem gesunden Gewebe und einem Tumorgewebe. Dieses unterschiedliche Verhalten kann mittels der Magnetresonanzbildgebung in einem Magnetresonanz-Elastogramm dargestellt werden und für eine Diagnose verwendet werden.

Bei einer Magnetresonanz-Elastographie-Untersuchung kann es dabei bei einer Generierung eines Antriebsmoments der Elastographievorrichtung zu unerwünschten Störungen und/oder Interferenzen mit einer Hochfrequenzantenneneinheit der Magnetresonanzvorrichtung kommen.

Wolfgang Loew et. al.: "A Dedicated 3 Tesla Prostate Coil for Magnetic Resonance Elastography, Imaging, and Tracking", Proceedings of the International Society for Magnetic Resonance in Medicine, ISMRM, Joint Annual Meeting ISMRM-ESMRMB, Milan, Italy, 10-16 May 2014, Nr 2180, 25. April 2014 (2014-04-25), XP040663249, offenbart ein System mit einer Magnetresonanzvorrichtung und einer Elastographievorrichtung, bei der Teilbereiche der Elastographievorrichtung in einem HFabgeschirmten Faraday-Käfig angeordnet sind.

Aus JP 2002 010991 A ist eine Vorrichtung bekannt, die eine schwingungserzeugende Vorrichtung (Elastographie-Vorrichtung) und eine Magnetresonanzvorrichtung umfasst.

Ein weitere Magnetresonanzvorrichtung mit einer Elastographievorrichtung ist beispielsweise in Qite Chen et. Al.: "The Design of Shear Wave Drive Device in Magnetic Resonance Elastography", Bioinformatics and Biomedical Engineering, (ICBBE) 2011 5th International Confernce, IEEE, 10. Mai 2011 (2011-05-10), Seiten 1-4, XP031878910, DOI: 10.1109/ICBBE.2011.5780456, ISBN: 978-1-4244-5088-6, beschrieben.

Aus US 2015/148663A1 ist ein Oszillationsapplikator für die MR-Rheologie bekannt. Der Oszillationsapplikator umfasst einen Schallkopf, der eine hin- und hergehende Bewegung bei einer bestimmten Frequenz erzeugt, und einen mechanisch mit dem Schallkopf gekoppelten Riemen, der um den Körper eines Patienten gewickelt werden kann.

Der vorliegenden Erfindung liegt insbesondere die Aufgabe zugrunde, eine Elastographievorrichtung anzugeben, die störungsfrei zusammen mit einer Magnetresonanzvorrichtung betrieben werden kann. Die Aufgabe wird durch die Merkmale des unabhängigen Anspruchs gelöst. Vorteilhafte Ausgestaltungen sind in den Unteransprüchen beschrieben.

Die Erfindung geht aus von einer Magnetresonanz-Elastographie-Vorrichtung mit einer Magnetresonanzvorrichtung und einer Elastographievorrichtung, wobei die Magnetresonanzvorrichtung umfasst:
- eine Scannereinheit,
- einen von der Scannereinheit zumindest teilweise umgebenen Patientenaufnahmebereich, und
- einen Patiententisch, der zu einem Einfahren eines Patienten in den Patientenaufnahmebereich ausgebildet ist,
wobei die Elastographievorrichtung umfasst:
- eine Antriebseinheit,
- einen Vibrationsapplikator, der für eine Magnetresonanz-Elastographie-Untersuchung am Patienten angeordnet ist, und
- eine Kraftübertragungseinheit, die zu einer Übertragung eines Antriebsmoments von der Antriebseinheit auf den Vibrationsapplikator ausgebildet ist.

Zudem umfasst die Elastographievorrichtung ein EMV-dichtes Schirmgehäuse mit einem zylinderförmigen Hochfrequenz-Hohlleiter (HF-Hohlleiter), wobei die Antriebseinheit innerhalb des Hochfrequenz-Hohlleiters angeordnet ist. Erfindungsgemäß umfasst der Hochfrequenz-Hohlleiter eine Länge und einen Durchmesser, wobei ein Verhältnis der Länge zu dem Durchmesser auf ein von Antriebseinheit erzeugtes Schwingungsspektrum so abgestimmt ist, dass eine Dämpfung von elektromagnetischen Wellen erreicht wird und damit auch eine unerwünschte Interaktion zwischen der Elastographievorrichtung und der Magnetresonanzvorrichtung verhindert wird.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Scannereinheit der Magnetresonanzvorrichtung umfasst bevorzugt eine Detektoreinheit, insbesondere eine Magneteinheit, zur Erfassung der medizinischen und/oder diagnostischen Bilddaten, insbesondere Magnetresonanzbilddaten. Hierbei umfasst die Scannereinheit, insbesondere die Magneteinheit, einen Grundmagneten, eine Gradientenspuleneinheit und eine Hochfrequenzantenneneinheit. Die Hochfrequenzantenneneinheit ist hierbei fest innerhalb der Scannereinheit angeordnet. Zudem kann die Magnetresonanzvorrichtung noch lokale Hochfrequenzspulen umfassen, die zur Erfassung von Magnetresonanzdaten um den zu untersuchenden Bereich eines Patienten angeordnet werden.

Der Grundmagnet ist zur Erzeugung eines homogenen Grundmagnetfelds mit einer definierten Magnetfeldstärke, wie beispielsweise mit einer Magnetfeldstärke von 0,55 T oder 1,5 T oder 3 T oder 7 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Für eine Magnetresonanzuntersuchung wird der Patient, insbesondere der zu untersuchende Bereich des Patienten, innerhalb eines Patientenaufnahmebereichs der Magnetresonanzvorrichtung positioniert. Der Patientenaufnahmebereich ist dabei zumindest teilweise von der Scannereinheit umgeben, insbesondere zylinderförmig von der Scannereinheit umgeben. Innerhalb des Patientenaufnahmebereichs ist bevorzugt ein Field of View (FOV) und/oder ein Isozentrum der Magnetresonanzvorrichtung angeordnet und/oder vorzufinden. Das FOV umfasst bevorzugt einen Erfassungsbereich der Magnetresonanzvorrichtung, innerhalb dessen die Bedingungen für eine Erfassung von medizinischen Bilddaten, insbesondere Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereichs weist die Magnetresonanzvorrichtung einen Patiententisch auf, der insbesondere innerhalb des Patientenaufnahmebereichs der Magnetresonanzvorrichtung bewegbar ausgebildet ist. Für eine Magnetresonanzuntersuchung wird der Patient zunächst auf dem Patiententisch der Patientenlagerungsvorrichtung positioniert und anschließend der Patiententisch zusammen mit dem Patienten in den Patientenaufnahmebereich eingefahren, bis der zu untersuchende Bereich des Patienten innerhalb des Isozentrums positioniert ist.

Die Antriebseinheit der Elastographievorrichtung ist zu einer Generierung eines Antriebsmoments, das bevorzugt an den Vibrationsapplikator übertragen wird, ausgebildet. Die Antriebseinheit kann hierzu eine Motoreinheit, beispielsweise einen Schrittmotor, aufweisen. Zudem kann die Antriebseinheit auch eine Motorsteuereinheit aufweisen.

Der Vibrationsapplikator der Elastographievorrichtung ist insbesondere zu einem Generieren von Vibrationen und/oder Druckwellen und zu einem Übertragen der generierten Vibrationen und/oder Druckwellen an den Patienten und/oder auf den Patienten ausgelegt. Bevorzugt wird hierbei der Vibrationsapplikator in einer Nähe zu dem Patienten, insbesondere in einer Nähe des zu untersuchenden Bereichs des Patienten, positioniert. Beispielsweise wird hierbei der Vibrationsapplikator an den zu untersuchenden Bereich des Patienten angelegt. Während einer Magnetresonanz-Elastographie-Untersuchung befindet sich somit der Vibrationsapplikator innerhalb des Patientenaufnahmebereichs, insbesondere innerhalb des FOVs.

Die Kraftübertragungseinheit weist ein Kraftübertragungselement auf, das bevorzugt magnetresonanzkompatibel ausgebildet ist, so dass während einer Magnetresonanz-Elastographie-Untersuchung keine Störungen von der Kraftübertragungseinheit hervorgerufen werden. Das Kraftübertragungselement kann dabei eine magnetresonanzkompatible Welle, insbesondere eine flexible Welle, umfassen, so dass einfache und flexibel Anordnung des Vibrationsapplikators bezüglich der Antriebseinheit möglich ist.

Das EMV(Elektro-Magnetische Verträglichkeit)-dichte Schirmgehäuse verhindert eine unerwünschte Interaktion zwischen Antriebseinheit und der Scannereinheit, insbesondere der Hochfrequenzantenneneinheit der Scannereinheit der Magnetresonanzvorrichtung. Ein EMV-dichtes Schirmgehäuse weist die Eigenschaft auf, unerwünschte elektrische oder elektromagnetische Effekte abzuschirmen. Insbesondere können derart elektrische oder elektromagnetische Effekte der Antriebseinheit der Elastographievorrichtung vorteilhaft abgeschirmt werden. Vorzugsweise umfasst der zylinderförmige HF-Hohlleiter einen kreisförmigen und/oder runden Querschnitt. Besonders vorteilhaft ist hierbei der HF-Hohlleiter bezüglich einer Längsachse, die bevorzugt durch eine Mitte des HF-Hohlleiter verläuft, rotationssymmetrisch ausgebildet.

Die Erfindung weist den Vorteil auf, dass die Elastographievorrichtung störungsfrei mit der Magnetresonanzvorrichtung für eine Magnetresonanz-Elastographie-Untersuchung betrieben werden kann. Dies ermöglicht auch eine einfache Integration der Elastographievorrichtung mit einer Magnetresonanzvorrichtung. Zudem kann die Antriebseinheit durch das EMV-dichte Schirmgehäuse an beliebigen Orten im Umfeld der Magnetresonanzvorrichtung positioniert werden. Des Weiteren weist diese Ausgestaltung der Erfindung den Vorteil auf, dass eine einfache geometrische Ausgestaltung des HF-Hohlleiters eine vorteilhafte Filterwirkung und/oder Abschirmung aufweist. Insbesondere kann mittels des HF-Hohlleiters hierbei eine vorteilhafte Dämpfung von elektromagnetischen Wellen erzielt werden und damit eine unerwünschte Interaktion zwischen der Elastographievorrichtung, insbesondere der Antriebseinheit der Elastographievorrichtung, und der Magnetresonanzvorrichtung verhindert werden.

In einer vorteilhaften Weiterbildung der erfindungsgemäßen Magnetresonanz-Elastographie-Vorrichtung kann es vorgesehen sein, dass die Antriebseinheit eine Motorsteuereinheit aufweist, wobei die Motorsteuereinheit innerhalb des EMV-dichten Schirmgehäuses angeordnet ist. Die Motorsteuereinheit ist bevorzugt zu einer Ansteuerung des Motors, beispielsweise eines Schrittmotors, der Elastographievorrichtung ausgebildet. Zudem kann die Motorsteuereinheit auch mit der Magnetresonanzvorrichtung gekoppelt sein, um einen Datenaustausch zwischen der Magnetresonanzvorrichtung und der Elastographievorrichtung zu ermöglichen, beispielsweise während einer Magnetresonanz-Elastographie-Untersuchung. Diese Ausgestaltung der Erfindung weist den Vorteil auf, dass eine besonders kompakte Anordnung der gesamten Antriebseinheit innerhalb des EMV-dichten Schirmgehäuses ermöglicht wird.

In einer vorteilhaften Weiterbildung der erfindungsgemäßen Magnetresonanz-Elastographie-Vorrichtung kann es vorgesehen sein, dass die Antriebseinheit zusammen mit dem EMV-dichten Schirmgehäuse außerhalb des Patientenaufnahmebereichs angeordnet ist. Insbesondere ist die Antriebseinheit zusammen mit dem EMV-dichten Schirmgehäuse während einer Magnetresonanz-Elastographie-Untersuchung außerhalb des Patientenaufnahmebereichs angeordnet. Dabei kann die Antriebseinheit zusammen mit dem EMV-dichten Schirmgehäuse beispielsweise an einem Fußende des Patiententischs angeordnet und/oder positioniert sein. Zudem kann die Antriebseinheit zusammen mit dem EMV-dichten Schirmgehäuse auch während einer Vorbereitung des Patienten und/oder der Magnetresonanz-Elastographie-Untersuchung neben dem Patiententisch angeordnet und/oder positioniert werden und dort auch während der Magnetresonanz-Elastographie-Untersuchung bleiben. Diese Ausgestaltung der Erfindung weist den Vorteil auf, dass während einer Magnetresonanz-Elastographie-Untersuchung genügend Platz für den Patienten innerhalb des Patientenaufnahmebereichs zur Verfügung steht.

Erfindungsgemäß umfasst der Hochfrequenz-Hohlleiter eine Länge und einen Durchmesser, wobei ein Verhältnis der Länge zu dem Durchmesser auf ein von der Antriebseinheit erzeugtes Schwingungsspektrum so abgestimmt ist, dass eine Dämpfung von elektromagnetischen Wellen erreicht wird und damit auch eine unerwünschte Interaktion zwischen der Elastographievorrichtung und der Magnetresonanzvorrichtung verhindert wird.

In einer vorteilhaften Weiterbildung der erfindungsgemäßen Magnetresonanz-Elastographie-Vorrichtung kann es vorgesehen sein, dass der Hochfrequenz-Hohlleiter ein nicht magnetisches, metallisches Rohr umfasst, wodurch eine vorteilhafte Abschirmung von elektromagnetischen Störeffekten erreicht werden kann. Bevorzugt umfasst der HF-Hohlleiter ein Rohr aus Aluminium.

In einer vorteilhaften Weiterbildung der erfindungsgemäßen Magnetresonanz-Elastographie-Vorrichtung kann es vorgesehen sein, dass der Hochfrequenz-Hohlleiter zwei Endbereiche aufweist, die in Längsrichtung des Hochfrequenz-Hohlleiters an gegenüberliegenden Seiten angeordnet sind, wobei zumindest ein Endbereich des Hochfrequenz-Hohlleiters offen ausgebildet ist. Dies ermöglicht eine konstruktiv einfache Abschirmung der Antriebseinheit. Zudem kann durch die offene Ausbildung des HF-Hohlleiters besonders einfach ein Kraftübertragungselement, beispielweise eine Antriebswelle, der Kraftübertragungseinheit von dem HF-Hohlleiter nach außen geleitet werden. Ist der HF-Hohlleiter an beiden Endbereichen offen ausgebildet, sollte die Antriebseinheit in einer Mitte des HF-Hohlleiters angeordnet sein, um eine effektive Dämpfung und/oder Abschirmung zu erreichen. Dabei sollte die Antriebseinheit mit einem gleichen Abstand von den beiden Endbereichen entfernt innerhalb des HF-Hohlleiters angeordnet sein. Ist dagegen der HF-Hohlleiter an nur einem Endbereich offen ausgebildet, kann eine Länge des HF-Hohlleiters gegenüber einer Ausbildung, die an beiden Endbereich offen ist, halbiert werden, um das gleiche Dämpfungsergebnis und/oder Abschirmungsergebnis zu erhalten, sofern die Antriebseinheit im Bereich des geschlossenen Endbereichs innerhalb des HF-Hohlleiters angeordnet ist. Der HF-Hohlleiter kann dabei eine Abdeckung umfassen, der den geschlossene Endbereich des HF-Hohlleiters abschließt. Dabei muss die Abdeckung fest mit dem Endbereich des HF-Hohlleiters verbunden sein, wie beispielsweise mit dem Endbereich verschweißt und/oder vernietet und/oder weiterer, dem Fachmann als sinnvoll erscheinender Befestigungen.

In einer vorteilhaften Weiterbildung der erfindungsgemäßen Magnetresonanz-Elastographie-Vorrichtung kann es vorgesehen sein, dass die Elastographievorrichtung eine Signalübertragungseinheit umfasst, um Signale zu der innerhalb des HF-Hohlleiters angeordneten Antriebseinheit zu übertragen, wobei die Signalübertragungseinheit zumindest ein optisches Faserkabel umfasst. Mittels der Signalübertragungseinheit können vorteilhaft Signale, insbesondere Datensignale, beispielsweise Steuersignale, und/oder eine Spannungsversorgung, für die Antriebseinheit und/oder für eine Stromversorgung der Antriebseinheit innerhalb des HF-Hohlleiters zur Verfügung gestellt werden. Bevorzugt ist hierbei die Signalübertragungseinheit derart ausgebildet, dass ein durch den HF-Hohlleiter erzeugter Filtereffekt und/oder Dämpfungseffekt durch Signalleitungen der Signalübertragungseinheit nicht beeinträchtigt wird sondern erhalten bleibt. Das zumindest eine optische Faserkabel kann beispielsweise ein Glasfaserkabel und/oder polymere Lichtwellenleiter usw. umfassen. Die Verwendung von optischen Faserkabeln weist den Vorteil auf, dass elektrisch nicht sichtbare Kabel zur Signalübertragung und/oder Datenübertragung verwendet werden können. Zudem kann derart verhindert werden, dass metallische Kabelteile in das EMV-dichte Schirmgehäuse, insbesondere in den HF-Hohlleiter, eingeführt werden und dabei beispielsweise als Antenne fungieren würden.

In einer vorteilhaften Weiterbildung der erfindungsgemäßen Magnetresonanz-Elastographie-Vorrichtung kann es vorgesehen sein, dass die Signalübertragungseinheit zumindest einen Durchgangsfilter und/oder Durchspeisefilters umfasst. Mittels des Durchgangsfilters kann vorteilhaft eine Stromversorgung der Antriebseinheit und/oder weiterer, innerhalb des EMV-dichten Schirmgehäuses, insbesondere innerhalb des HF-Hohlleiters, angeordneter Einheiten, bereitgestellt werden. Bevorzugt ist der zumindest eine Durchgangsfilter mit Kontakt zu dem HF-Hohlleiter innerhalb des HF-Hohlleiters angeordnet, beispielsweise über eine gemeinsame Kontaktfläche.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße Magnetresonanz-Elastographie-Vorrichtung mit einer Magnetresonanzvorrichtung und einer Elastographievorrichtung in einer schematischen Darstellung und
- Fig. 2: ein erstes Ausführungsbeispiel eines HF-Hohlleiters der Elastographievorrichtung, und
- Fig. 3: ein zweites Ausführungsbeispiel eines HF-Hohlleiters der Elastographievorrichtung.

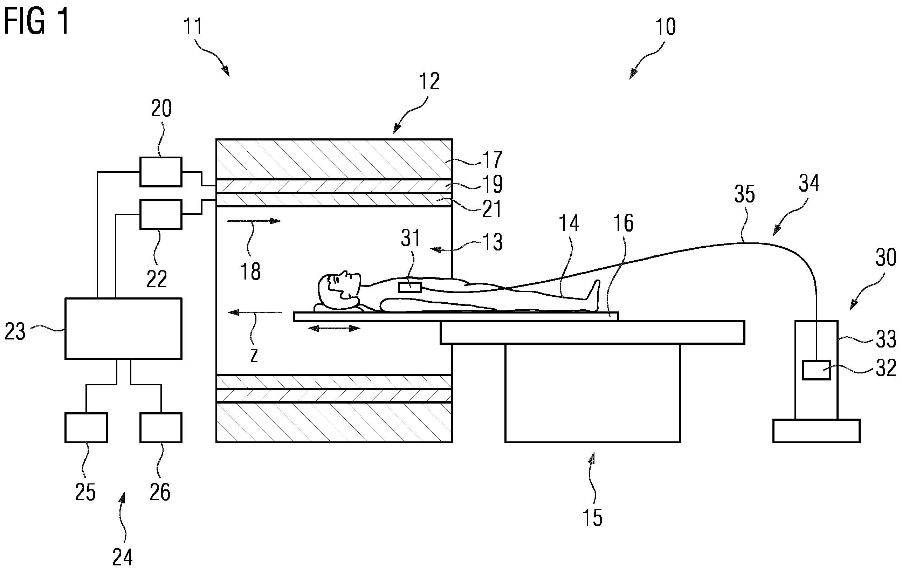

In Fig. 1 ist eine Magnetresonanz-Elastographie-Vorrichtung 10 schematisch dargestellt. Die Magnetresonanz-Elastographie-Vorrichtung 10 umfasst eine Magnetresonanzvorrichtung 11 und eine Elastographievorrichtung 30.

Die Magnetresonanzvorrichtung 11 umfasst eine von einer Magneteinheit gebildeten Scannereinheit 12. Zudem weist die Magnetresonanzvorrichtung 11 einen Patientenaufnahmebereich 13 auf zu einer Aufnahme eines Patienten 14. Der Patientenaufnahmebereich 13 im vorliegenden Ausführungsbeispiel ist zylinderförmig ausgebildet und in einer Umfangsrichtung von der Scannereinheit 12, insbesondere von der Magneteinheit, zylinderförmig umgeben. Grundsätzlich ist jedoch eine davon abweichende Ausbildung des Patientenaufnahmebereichs 13 jederzeit denkbar. Der Patient 14 kann mittels einer Patientenlagerungsvorrichtung 15 der Magnetresonanzvorrichtung 11 in den Patientenaufnahmebereich 13 geschoben und/oder gefahren werden. Die Patientenlagerungsvorrichtung 15 weist hierzu einen innerhalb des Patientenaufnahmebereichs 13 bewegbar ausgestalteten Patiententisch 16 auf. Insbesondere ist hierbei der Patiententisch 16 in Richtung einer Längserstreckung des Patientenaufnahmebereichs 13 und/oder in z-Richtung bewegbar gelagert.

Die Scannereinheit 12, insbesondere die Magneteinheit, umfasst einen supraleitenden Grundmagneten 17 zu einem Erzeugen eines starken und insbesondere konstanten Grundmagnetfelds 18. Weiterhin weist die Scannereinheit 12, insbesondere die Magneteinheit, eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 20 der Magnetresonanzvorrichtung 11 gesteuert. Die Scannereinheit 12, insbesondere die Magneteinheit, umfasst weiterhin eine Hochfrequenzantenneneinheit 21 zu einer Anregung einer Polarisation, die sich in dem von dem Grundmagneten 17 erzeugten Grundmagnetfeld 18 einstellt. Die Hochfrequenzantenneneinheit 21 wird von einer Hochfrequenzantennensteuereinheit 22 der Magnetresonanzvorrichtung 11 gesteuert und strahlt hochfrequente Magnetresonanzsequenzen in den Patientenaufnahmebereich 13 der Magnetresonanzvorrichtung 11 ein.

Zu einer Steuerung des Grundmagneten 17, der Gradientensteuereinheit 19 und zur Steuerung der Hochfrequenzantennensteuereinheit 21 weist die Magnetresonanzvorrichtung 11 eine Systemsteuereinheit 23 auf. Die Systemsteuereinheit 23 steuert zentral die Magnetresonanzvorrichtung 11, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 11 eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Ausgabeeinheit 25, beispielsweise auf zumindest einem Monitor, der Benutzerschnittstelle 24 für ein medizinisches Bedienpersonal angezeigt und/oder ausgegeben werden. Weiterhin weist die Benutzerschnittstelle 24 eine Eingabeeinheit 26 auf, mittels der Informationen und/oder Parameter während eines Messvorgangs von dem medizinischen Bedienpersonal eingegeben werden können.

Die Elastographievorrichtung 30 umfasst einen Vibrationsapplikator 31, der eine Vibrationserzeugereinheit aufweist. Der Vibrationsapplikator 31 ist während einer Magnetresonanz-Elastographie-Untersuchung direkt am Patienten 14, insbesondere an dem zu untersuchenden Bereich des Patienten 14, angeordnet.

Des Weiteren weist die Elastographievorrichtung 30 eine Antriebseinheit 32 auf. Die Antriebseinheit 32 ist dazu ausgelegt und/oder ausgebildet, ein Antriebsmoment für die Vibrationserzeugereinheit während einer Elastographie-Untersuchung zu erzeugen und/oder zu generieren. Um Störungen und/oder unterwünschte Interaktionen zwischen der Antriebseinheit 32 und der Scannereinheit 12, insbesondere der Hochfrequenzantenneneinheit 21, zu vermeiden, weist die Elastographievorrichtung 30 zudem ein EMV-dichtes Schirmgehäuse 33 auf, in dem die Antriebseinheit 32 angeordnet ist. Die Antriebseinheit 32 ist während einer Magnetresonanz-Elastographie-Untersuchung zusammen mit dem EMV-dichten Schirmgehäuse 33 außerhalb des Patientenaufnahmebereichs 13 der Magnetresonanzvorrichtung 11 angeordnet.

Zu einer Übertragung eines Antriebsmoments von der Antriebseinheit 32 auf den Vibrationsapplikator 31 weist die Elastographievorrichtung 30 eine Kraftübertragungseinheit 34 auf. Die Kraftübertragungseinheit 34 weist zumindest ein Kraftübertragungselement 35 auf, das bevorzugt als flexible und magnetresonanzkompatible Welle ausgebildet ist.

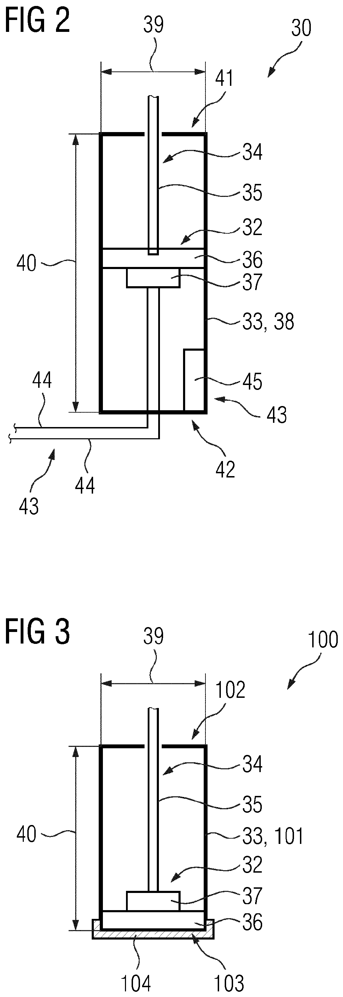

In Fig. 2 ist ein erstes Ausführungsbeispiel der Elastographievorrichtung 30, insbesondere der Antriebseinheit 32 und des EMV-dichten Schirmgehäuses 33 der Elastographievorrichtung 30, näher dargestellt. Die Antriebseinheit 32 umfasst eine Motoreinheit 36, die beispielsweise als Schrittmotor ausgebildet ist, und eine Motorsteuereinheit 37. Sowohl die Motoreinheit 36 als auch die Motorsteuereinheit 37 sind dabei innerhalb des EMV-dichten Schirmgehäuses 33 angeordnet.

Das EMV-dichte Schirmgehäuse 33 umfasst eine zylinderförmigen Hochfrequenz-Hohlleiter (HF-Hohlleiter 38). Der HF-Hohlleiter 38 umfasst ein nichtmagnetisches Rohr. Bevorzugt weist der HF-Hohlleiter 38, insbesondere das Rohr, einen runden und/oder kreisförmigen Querschnitt auf. Der HF-Hohlleiter 38 umfasst zudem ein metallisches Rohr, wie beispielsweise ein Rohr aus Aluminium.

Um eine vorteilhafte Abschirmung und/oder Dämpfung zu erreichen, weist der HF-Hohlleiter 38 einen Durchmesser 39 und eine Länge 40 auf, wobei ein Verhältnis der Länge 40 zu dem Durchmesser 39 des HF-Hohlleiters 38 auf ein von der Antriebseinheit 32 erzeugtes Schwingungsspektrum abgestimmt ist.

Der HF-Hohlleiter 38 weist zudem zwei Endbereiche 41, 42 auf, die in Längsrichtung des HF-Hohlleiters 38 an gegenüberliegenden Seiten und/oder Enden angeordnet sind. Im vorliegenden Ausführungsbeispiel sind beide Endbereiche 41, 42 des HF-Hohlleiters 38 offen ausgebildet. In diesem Ausführungsbeispiel ist bevorzugt die Antriebseinheit 32 in einer Mitte des HF-Hohlleiters 38, insbesondere in Längsrichtung des HF-Hohlleiters 38 in einer Mitte, angeordnet, so dass zu beiden Endbereichen 41, 42 des HF-Hohlleiters 38 ein möglichst gleicher Abstand vorhanden ist, um eine effektive Dämpfung und/oder Abschirmung von elektromagnetischen Wellen zu gewährleisten. Durch einen ersten Endbereich 41 der beiden Endbereiche 41, 42 ist das Kraftübertragungselement 35, insbesondere die Antriebswelle, von dem HF-Hohlleiter 38 nach außen geführt.

Die Elastographievorrichtung 30 weist des Weiteren eine Signalübertragung 43 auf, um Signale zu der innerhalb des HF-Hohlleiters 38 angeordneten Antriebseinheit 32, insbesondere der Motoreinheit 36 und der Motorsteuereinheit 37, zu übertragen. Mittels der Signalübertragungseinheit 43 können vorteilhaft Signale, insbesondere Datensignale, beispielsweise Steuersignale, und/oder eine Spannungsversorgung, für die Antriebseinheit 32 und/oder für eine Stromversorgung der Antriebseinheit 32 zur Verfügung gestellt werden. Die Signalübertragungseinheit 43 umfasst dabei zumindest ein optisches Faserkabel 44. Das zumindest eine optische Faserkabel 44 kann beispielsweise ein Glasfaserkabel und/oder polymere Lichtwellenleiter usw. umfassen. Das zumindest eine optische Faserkabel 44 ist für eine Datenübertragung und/oder eine Spannungsversorgung ausgelegt. Die Signalübertragungseinheit 43 kann auch mehr als ein optisches Faserkabel 44 zur Datenübertragung und/oder zur Spannungsversorgung der Antriebseinheit 32 aufweisen. Beispielsweise können für eine Spannungsversorgung der Antriebseinheit 32 zwei optische Faserkabel 44 zur Verfügung stehen.

Des Weiteren weist die Signalübertragungseinheit 43 zumindest einen Durchgangsfilter 45 und/oder einen Durchspeisefilter auf für eine Stromversorgung, insbesondere eine Stromversorgung der Antriebseinheit 32, insbesondere der Motoreinheit 36. Der Durchgangsfilter 45 und/oder der Durchspeisefilter ist dabei mit Kontakt zu dem HF-Hohlleiter 38 innerhalb des HF-Hohlleiters 38 angeordnet. Im vorliegenden Ausführungsbeispiel weist der Durchgangsfilter 45 und/oder der Durchspeisefilter eine Kontaktfläche mit dem HF-Hohlleiter 38 auf.

In Fig. 3 ist ein alternatives Ausführungsbeispiel der Elastographievorrichtung 100, insbesondere eines HF-Hohlleiters 101 der Elastographievorrichtung 100, dargestellt. Im Wesentlichen gleichbleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in den Fig. 1 und 2, wobei bezüglich gleichbleibender Bauteile, Merkmale und Funktionen auf die Beschreibung des Ausführungsbeispiels in den Fig. 1 und 2 verwiesen wird.

Die Elastographievorrichtung 100 in Fig. 3 unterscheidet sich von der Elastographievorrichtung 100 in Fig. 2 in einer Ausbildung des HF-Hohlleiters 101. Eine weitere Ausgestaltung der Elastographievorrichtung 100 entspricht den zu Fig. 2 dargestellten und beschriebenen Ausführungen.

Die Elastographievorrichtung 100 weist ebenfalls einen HF-Hohlleiter 101, innerhalb dessen die Antriebseinheit 32 angeordnet ist. Der HF-Hohlleiter 101 weist zwei Endbereiche 102, 103 auf, die in Längsrichtung des HF-Hohlleiters 101 an gegenüberliegenden Seiten und/oder Enden angeordnet sind. Im vorliegenden Ausführungsbeispiel ist nur ein Endbereich 102 der beiden Endbereiche 102, 103 des HF-Hohlleiters 101 offen ausgebildet. Durch den offenen Endbereich 102 ist dabei das Kraftübertragungselement 35, insbesondere die Welle, von dem HF-Hohlleiter 101 nach außen geführt. Der Weitere Endbereich 103 der beiden Endbereiche 102, 103 ist dagegen geschlossen ausgebildet. Dabei weist der geschlossen Endbereich 103 eine Abdeckung 104 auf, die fest mit dem Endbereich 103 des HF-Hohlleiters 101 verbunden ist, wie beispielsweise mit dem Endbereich 103 verschweißt und/oder vernietet und/oder durch weitere, dem Fachmann als sinnvoll erscheinender Befestigungsarten mit dem Endbereich 103 befestigt ist.

Bei einer derartigen Ausgestaltung des HF-Hohlleiters 101 kann die Antriebseinheit 32, insbesondere die Motoreinheit 36 und die Motorsteuereinheit 37, in diesem geschlossenen Endbereich 103 des HF-Hohlleiters 101 angeordnet sein. Dies ermöglicht auch, dass der HF-Hohlleiter 101 nur in eine Richtung die für eine Abschirmung und/oder Dämpfung von elektromagnetischen Wellen erforderliche Länge aufweisen muss.

Die weitere Ausgestaltung des HF-Hohlleiters 101 und auch der Signalübertragungseinheit 43 zur Übertragung von Signalen und/oder Daten in den HF-Hohlleiter 101 entspricht dabei den Ausführungen zur Fig. 2, auf die hiermit verwiesen wird.

Die in den Fig. 1 bis 3 dargestellten Magnetresonanz-Elastographie-Vorrichtungen 10 können selbstverständlich weitere Komponenten umfassen, die Magnetresonanz-Elastographie-Vorrichtungen 10 gewöhnlich aufweisen. Eine allgemeine Funktionsweise einer Magnetresonanz-Elastographie-Vorrichtung 10 ist zudem dem Fachmann bekannt, so dass auf eine detaillierte Beschreibung der weiteren Komponenten verzichtet wird.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanz-Elastographie-Vorrichtung (10) mit einer Magnetresonanzvorrichtung (11) und einer Elastographievorrichtung (30, 100), wobei die Magnetresonanzvorrichtung (11) umfasst:
- eine Scannereinheit (12),
- einen von der Scannereinheit (12) zumindest teilweise umgebenden Patientenaufnahmebereich (13), und
- einen Patiententisch (16), der zu einem Einfahren eines Patienten (14) in den Patientenaufnahmebereich (13) ausgebildet ist,
wobei die Elastographievorrichtung (30, 100) umfasst:
- eine Antriebseinheit (32),
- eine Vibrationsapplikator (31), der für eine Magnetresonanz-Elastographie-Untersuchung am Patienten (14) angeordnet ist, und
- eine Kraftübertragungseinheit (34), die zu einer Übertragung eines Antriebsmoments von der Antriebseinheit (32) auf den Vibrationsapplikator (31) ausgebildet ist,
wobei die Elastographievorrichtung ein EMV-dichtes Schirmgehäuse (33) mit einem zylinderförmigen Hochfrequenz-Hohlleiter (38, 101) umfasst, wobei die Antriebseinheit (32) innerhalb des Hochfrequenz-Hohlleiters (38, 101) angeordnet ist,
wobei der Hochfrequenz-Hohlleiter (38, 101) eine Länge (40) und einen Durchmesser (39) umfasst, **dadurch gekennzeichnet, dass** ein Verhältnis der Länge (40) zu dem Durchmesser (39) auf ein von der Antriebseinheit (32) erzeugtes Schwingungsspektrum so abgestimmt ist, dass eine Dämpfung von elektromagnetischen Wellen erreicht wird und damit auch eine unerwünschte Interaktion zwischen der Elastographievorrichtung und der Magnetresonanzvorrichtung verhindert wird.

2. Magnetresonanz-Elastographie-Vorrichtung (10) nach Anspruch 1,
**dadurch gekennzeichnet, dass** die Antriebseinheit (32) eine Motorsteuereinheit (37) aufweist, wobei die Motorsteuereinheit (37) innerhalb des EMV-dichten Schirmgehäuses (33) angeordnet ist.

3. Magnetresonanz-Elastographie-Vorrichtung (10) nach einem der vorhergehenden Ansprüche,
**dadurch gekennzeichnet, dass** die Antriebseinheit (32) zusammen mit dem EMV-dichten Schirmgehäuse (33) außerhalb des Patientenaufnahmebereichs (13) angeordnet ist.

4. Magnetresonanz-Elastographie-Vorrichtung (10) nach einem der vorhergehenden Ansprüche,
**dadurch gekennzeichnet, dass** der Hochfrequenz-Hohlleiter (38, 101) ein nicht magnetisches, metallisches Rohr umfasst.

5. Magnetresonanz-Elastographie-Vorrichtung (10) nach einem der vorhergehenden Ansprüche,
**dadurch gekennzeichnet, dass** der Hochfrequenz-Hohlleiter (38, 101) zwei Endbereiche (41, 42, 102, 103) aufweist, die in Längsrichtung des Hochfrequenz-Hohlleiters (38, 101) an gegenüberliegenden Seiten angeordnet sind, wobei zumindest ein Endbereich (41, 42, 102) des Hochfrequenz-Hohlleiters (38, 101) offen ausgebildet ist.

6. Magnetresonanz-Elastographie-Vorrichtung (10) nach einem der vorhergehenden Ansprüche,
**dadurch gekennzeichnet, dass** die Elastographievorrichtung (30, 100) eine Signalübertragungseinheit (43) umfasst, um Signale zu der innerhalb des Hochfrequenz-Hohlleiters (38, 101) angeordneten Antriebseinheit (32) zu übertragen, wobei die Signalübertragungseinheit (43) zumindest ein optisches Faserkabel (44) umfasst.

7. Magnetresonanz-Elastographie-Vorrichtung (10) nach Anspruch 6,
**dadurch gekennzeichnet, dass** die Signalübertragungseinheit (43) zumindest einen Durchgangsfilter (45) umfasst.

8. Magnetresonanz-Elastographie-Vorrichtung (10) nach
Anspruch 7,
**dadurch gekennzeichnet, dass** der zumindest eine Durchgangsfilter (45) mit Kontakt zu dem Hochfrequenz-Hohlleiter (38, 101) innerhalb des Hochfrequenz-Hohlleiters (38, 101) angeordnet ist.

## Claims

1. Magnetic resonance elastography apparatus (10) with a magnetic resonance apparatus (11) and an elastography apparatus (30, 100), wherein the magnetic resonance apparatus (11) comprises:
- a scanner unit (12),
- a patient receiving region (13) surrounded at least partially by the scanner unit (12), and
- a patient couch (16), which is embodied to introduce a patient (14) into the patient receiving region (13),
wherein the elastography apparatus (30, 100) comprises:
- a drive unit (32),
- a vibration applicator (31), which is arranged on the patient (14) for a magnetic resonance elastography examination, and
- a force transmission unit (34), which is embodied to transmit a drive moment from the drive unit (32) to the vibration applicator (31),
wherein the elastography apparatus comprises an EMC-tight shield housing (33) with a cylindrical radiofrequency hollow conductor (38, 101), wherein the drive unit (32) is arranged within the radiofrequency hollow conductor (38, 101),
wherein the radiofrequency hollow conductor (38, 101) comprises a length (40) and a diameter (39), **characterised in that** a ratio of the length (40) to the diameter (39) is matched to an oscillation spectrum produced by the drive unit (32) so that an attenuation of electromagnetic waves is achieved and an unwanted interaction between the elastography apparatus and the magnetic resonance apparatus is also prevented.

2. Magnetic resonance elastography apparatus (10) according to claim 1,
**characterised in that** the drive unit (32) has a motor control unit (37), wherein the motor control unit (37) is arranged within the EMC-tight shield housing (33).

3. Magnetic resonance elastography apparatus (10) according to one of the preceding claims,
**characterised in that** the drive unit (32) together with the EMC-tight shield housing (33) is arranged outside of the patient receiving region (13).

4. Magnetic resonance elastography apparatus (10) according to one of the preceding claims,
**characterised in that** the radiofrequency hollow conductor (38, 101) comprises a non-magnetic, metallic tube.

5. Magnetic resonance elastography apparatus (10) according to one of the preceding claims,
**characterised in that** the radiofrequency hollow conductor (38, 101) has two end regions (41, 42, 102, 103), which are arranged in the longitudinal direction of the radiofrequency hollow conductor (38, 101) on opposite sides, wherein at least one end region (41, 42, 102) of the radiofrequency hollow conductor (38, 101) is embodied to be open.

6. Magnetic resonance elastography apparatus (10) according to one of the preceding claims,
**characterised in that** the elastography apparatus (30, 100) comprises a signal transmission unit (43), in order to transmit signals to the drive unit (32) arranged within the radiofrequency hollow conductor (38, 101), wherein the signal transmission unit (43) comprises at least one fiber optic cable (44).

7. Magnetic resonance elastography apparatus (10) according to claim 6,
**characterised in that** the signal transmission unit (43) comprises at least one through filter (45).

8. Magnetic resonance elastography apparatus (10) according to claim 7,
**characterised in that** the at least one through filter (45) is arranged in contact with the radiofrequency hollow conductor (38, 101) within the radiofrequency hollow conductor (38, 101).

## Revendications

1. Dispositif (10) d'élastographie par résonance magnétique, comprenant un dispositif (11) de résonance magnétique et un dispositif (30, 100) d'élastographie, dans lequel le dispositif (11) de résonance magnétique comprend :
- une unité (12) de scanner,
- une zone (13) d'enregistrement de patient entourée au moins en partie par l'unité (12) de scanner, et
- une table (16) de patient, qui est constituée pour l'entrée d'un patient (14) dans la zone (13) d'enregistrement d'un patient,
dans lequel le dispositif (30, 100) d'élastographie comprend :
- une unité (32) d'entraînement,
- un applicateur (31) de vibrations, qui est disposé sur le patient (14) pour une recherche d'élastographie par résonance magnétique, et
- une unité (34) de transmission de force, qui est constituée pour la transmission d'un couple d'entraînement de l'unité (32) d'entraînement à l'applicateur (31) de vibrations,
dans lequel le dispositif d'élastographie comprend un carter (33) de blindage étanche à compatibilité à l'électromagnétisme ayant un conducteur (38, 101) creux de haute fréquence de forme cylindrique,
dans lequel l'unité (32) d'entraînement est disposée à l'intérieur du conducteur (38, 101) creux à haute fréquence,
dans lequel le conducteur (38, 101) creux à haute fréquence a une longueur (40) et un diamètre (39),
**caractérisé en ce qu'**
un rapport de la longueur (40) au diamètre (39) est adapté à un spectre de vibrations produit par l'unité (32) d'entraînement, de manière à obtenir un amortissement d'ondes électromagnétiques et à empêcher ainsi également une interaction non souhaitée entre le dispositif d'élastographie et le dispositif de résonance magnétique.

2. Dispositif (10) d'élastographie par résonance magnétique suivant la revendication 1,
**caractérisé en ce que** l'unité (32) d'entraînement a une unité (37) de commande de moteur, dans lequel l'unité (37) de commande de moteur est disposée à l'intérieur du carter (33) de blindage étanche à compatibilité à l'électromagnétisme.

3. Dispositif (10) d'élastographie par résonance magnétique suivant l'une des revendications précédentes,
**caractérisé en ce que** l'unité (32) d'entraînement est disposée ensemble avec le carter (33) de blindage étanche à compatibilité à l'électromagnétisme à l'extérieur de la zone (13) d'enregistrement de patient.

4. Dispositif (10) d'élastographie par résonance magnétique suivant l'une des revendications précédentes,
**caractérisé en ce que** le conducteur (38, 101) creux à haute fréquence comprend un tube métallique amagnétique.

5. Dispositif (10) d'élastographie par résonance magnétique suivant l'une des revendications précédentes,
**caractérisé en ce que** le conducteur (38, 101) creux à haute fréquence a deux tronçons (41, 42, 102, 103) d'extrémité, qui sont disposés dans la direction longitudinale du conducteur (38, 101) creux à haute fréquence sur des côtés opposés, dans lequel au moins un tronçon (41, 42, 102) d'extrémité du conducteur (38, 101) creux à haute fréquence est de constitution ouverte.

6. Dispositif (10) d'élastographie par résonance magnétique suivant l'une des revendications précédentes,
**caractérisé en ce que** le dispositif (30, 100) d'élastographie comprend une unité (43) de transmission du signal afin de transmettre des signaux à l'unité (32) d'entraînement disposée à l'intérieur du conducteur (38, 101) creux à haute fréquence, dans lequel l'unité (43) de transmission de signal comprend au moins un câble (44) à fibre optique.

7. Dispositif (10) d'élastographie par résonance magnétique suivant la revendication 6,
**caractérisé en ce que** l'unité (43) de transmission du signal comprend au moins un filtre (45) de passage.

8. Dispositif (10) d'élastographie par résonance magnétique suivant la revendication 7,
**caractérisé en ce que** le au moins un filtre (45) de passage est monté avec contact avec le conducteur (38, 101) creux à haute fréquence à l'intérieur du conducteur (38, 101) creux à haute fréquence.
